# EUROPÄISCHE PATENTSCHRIFT

(11) **EP 1 355 148 B1**
(45) Veröffentlichungstag und Bekanntmachung des Hinweises auf die Patenterteilung: **05.10.2005**
(21) Anmeldenummer: 03004626.2
(22) Anmeldetag: 03.03.2003
(51) Int. Cl.: G01N 33/46, G01N 21/88, G01B 11/30, G01N 21/89

(54) **Verfahren zum Erkennen der Anwesenheit von Fehlern, wie Rissen oder abgefasten Rändern, auf der Oberfläche von Holzbrettern**
Method to assess the presence of defects, such as cracks or bevelled edges, on the surface of wooden boards
Procédure pour la détection des défectuosités, telles que fêlures ou bords chanfreinés sur la surface de planches de bois

(30) Priorität: 18.04.2002 IT BZ20020018
(43) Veröffentlichungstag der Anmeldung: 22.10.2003
(73) Patentinhaber: MICROTEC S.r.l., 39042 Bressanone (Bolzano) (IT)
(72) Erfinder: Giudiceandrea, Federico, 39042 Bressanone (BZ) (IT)
(74) Vertreter: Ghioni, Carlo Raoul Maria

(56) Entgegenhaltungen:
- EP-A- 0 692 714
- EP-A- 0 787 970
- WO-A-93/22659
- WO-A-95/24636
- US-A- 4 984 172
- ASTRAND E ET AL: "A SINGLE CHIP MULTI-FUNCTION SENSOR SYSTEM FOR WOOD INSPECTION" PROCEEDINGS OF THE IAPR INTERNATIONAL CONFERENCE ON PATTERN RECOGNITION. JERUSALEM, OCT. 9 - 13, 1994. CONFERENCE C: SIGNAL PROCESSING / CONFERENCE D: PARALLEL COMPUTING, LOS ALAMITOS, IEEE COMP. SOC. PRESS, US, Bd. 3 CONF. 12, 9. Oktober 1994 (1994-10-09), Seiten 300-304, XP000509968 ISBN: 0-8186-6277-8

## Beschreibung

Die Erfindung betrifft ein Verfahren zum Erkennen der Anwesenheit von Fehlern, wie Rissen oder abgefasten Ränder an der Oberfläche von Holzbrettern, gemäß dem Oberbegriff des Anspruches 1.

Risse und abgefasten Ränder sind nicht die einzigen Fehler, die ein Holzbrett aufweisen kann. Weitere Fehler, die ein Holzbrett aufweisen kann, sind Knoten, deren Form und deren Farbe verschiedenartig sein können, und die Harzeinschlüsse. Es ist schon bekannt die Anwesenheit dieser letzteren Fehler, d.h. Knoten oder Harzeinschlüsse, in Holzbrettern zu erkennen, indem die Oberfläche des Brettes mit Lichtbündeln beaufschlagt wird, und dann die nachfolgende Erscheinung von Lichtreflexion und/oder Lichtstreuung untersucht wird.

Bezüglich dieser beiden Erscheinungen wird kurz gefasst erinnert, dass in Anwesenheit einer idealen Oberfläche, der sogenannten Spiegelfläche, für jede Stelle dieser Oberfläche eine gerichtete Reflexion erhalten wird, gemäß welcher der einfallende Lichtstrahl ganz längs der sogenannten gerichtetetn Reflexionsrichtung mit einem Winkel zurückgestrahlt wird, der dem Einfallwinkel gleich, jedoch zu diesem entgegengerichtet ist. In Anwesenheit von Oberflächen, die nicht als Spiegelflächen betrachtet werden können, und dies ist die Mehrzahl, ist die Reflexion gemischt, d.h. ein Teil des einfallenden Strahls wird wieder längs der gerichteten Reflexionsrichtung zurückgestrahlt, während der restliche Teil des einfallenden Winkels zerstreut wird, d.h. er wird mehr oder weniger stark und regelmäßig um die gerichetete Reflexionsrichtung abgelenkt, ohne dem oben genannten für die Spiegelflächen geltende geometrische Gesetz der Reflexion zu unterliegen.

Wenn dann die Oberfläche keine Spiegelfläche ist und zudem auch nicht einmal gleichförmig ist, wie beispielsweise jene der Holzbretter, die Knoten oder Harzeinschlüsse aufweisen, wird eine gemischte Reflexion vorliegen, die von Mal zu Mal verschieden sein wird, je nachdem, ob die Stelle zu dem Knoten, zu dem Harzeinschluss oder zu der restlichen Oberfläche des Holzbrettes angehört. Die Verschiedenheit der erfassten, gemischten Reflexion erlaubt also die dem Knoten angehörenden Stellen von jenen die dem Harzeinschluss angehören und von jenen die der restlichen Oberfläche des Holzbrettes angehören zu unterscheiden.

Aus dem Patent EP 0 580 909 ist es bekannt, die längs der gerichteten Reflexionsrichtung rückgestrahlte Lichtstärke und die längs der Einfallsrichtung des Lichtstrahles zerstreute Lichtstärke Stelle für Stelle der Oberfläche des Holzbrettes zu vergleichen, um auszusagen, dass die Gegenstand der Untersuchung bildende Stelle einem Knoten oder Harzeinschluss angehört, wenn die zurückgestrahlte Lichtstärke und die zerstreute Lichtstärke etwa gleich sind, und um hingegen auszusagen, dass an der Gegenstand der Untersuchung bildenden Stelle keine Knoten oder Harzeinschlüsse vorliegen, wenn die rückgestrahlte Lichtstärke sehr viel größer ist als die zerstreute.

Aus dem Patent US 4,606,645 ist es bekannt die Anwesenheit von Knoten zu bestimmen, indem die Tatsache ausgenutzt wird, dass deren Fasern senkrecht zu den restlichen Fasern des Holzbrettes ausgerichtet sind. Dieses Patent schlägt vor die Ausrichtung der das Holz bildenden Fasern zu ermitteln, indem im oberhalb des Holzbrettes liegenden Raum die Anordnung der Ströme des zurückgestrahlten und des zerstreuten Lichtes untersucht werden. Zu diesem Zwecke sind Geräte zum Erfassen der Lichtstärke vorgesehen, die längs eines Kreises angeordnet sind, durch dessen Mitte der einfallende Lichtstrahl läuft.

Keines der beiden in diesen Dokumente beschriebenen Verfahren ist geeignet außer den Knoten und den Harzeinschlüssen auch die Risse und die schlecht geschnittenen, d.h. abgefasten Ränder, zu erkennen. In diesen Dokumenten wird nicht einmal auf diese Möglichkeit hingewiesen.

Aus dem Patent EP 0 692 714 ist ein Verfahren bekannt zum Feststellen der geometrischen Merkmale von parallelflachen Gegenständen. In diesem Verfahren werden verschiedene Seiten des Gegenstandes mit verschiedenen Lichtbündeln beleuchtet, und zwar wird die seitliche Seite mit einem monochromatischen Licht beleuchtet, während die oben liegende Seite mit einem mehrchromatischen Licht beleuchtet wird. Das reflektierte Licht wird von einer vorgesehenen Kamera erfasst. Danach werden in Abhängigkeit des jeweiligen Pixels, der das reflektierte Licht einer Stelle erfasst hat, und der Zusammensetzung dieses erfassten Lichtes Schlussfolgerungen gezogen über die Zugehörigkeit der Stelle zu einer der beiden Seiten und über derer Lage.

Aus dem Patent WO9524636A sind eine Vorrichtung und ein Verfahren bekannt zur Erkennung von Fehlern in dem Holz. Insbesondere betrifft dieses Patent mehrere Ausführungformen, von denen insbesondere zwei zu erwähnen sind.

Die erste davon sieht im Allgemeinen vor, dass man den Gegenstand von zwei gegenüberliegenden Seiten mit zwei koplanaren ebenen Lichtbündeln beleuchetet, die auf derselben Öberflächenlinie einfallen, und dann das reflektierte und das infolge des sogenannten Tracheid-Effekts in dem Holz zerstreute Licht mit einer oder auch zwei Kameras erfasst werden. Dabei wird der Gegenstand zugleich von beiden Seiten beleuchtet, denn der Sinn dieser zugleichen Beleuchtung besteht darin, möglichst alle Stellen der Linie zu beleuchten, damit jede Stelle das Licht reflektieren kann und darüberhinaus damit in jeder Stelle das Tracheid-Effekt stattfinden kann. Werden zwei Kameras vorgesehen, ist die eine der Erfassung des reflektierten Lichtes gewidmet, während die andere der Erfassung des infolge des Tracheid-Effekts zertreuten Lichtes gewidmet ist. Es werden die Intensität des reflektierten Lichtes und des infolge des Tracheid-Effekts in dem Holz zerstreuten Lichtes verglichen und daraus werden mit Hilfe von Bildbearbeitungsverfahren, über die aber nichts Näheres bekannt gegeben wird, Schlussfolgerungen über das Vorhandensein von Fehlern in dem Holz gezogen.

Die zweite Ausführungsform sieht vor, dass, man den Gegenstand wiederum von zwei gegenüberliegenden Seiten beleuchtet, aber diesmal mit zwei parallelen ebenen Lichtbündeln, die auf zwei verschiedene gleichenfalls parallele und nahe liegende Oberflächenlinien einfallen. Diesmal wird nur das von den beiden Linien reflektierte Licht mit einer oder auch zwei Kameras erfasst.

Dabei wird wiederum der Gegenstand zugleich von beiden Seiten beleuchtet. Werden zwei Kameras vorgesehen, ist die eine der Erfassung des reflektierten Lichtes von einer Linie gewidmet, während die andere der Erfassung des reflektierten Lichtes der anderen naheliegenden Linie gewidmet ist. Es werden die Intensitäten des reflektierten Lichtes der beiden Linien verglichen, um daraus Schlussfolgerungen über das Vorhandensein von Fehlern in dem Holz zu ziehen, aber es wird nicht angegeben wie dies geschehen soll.

Aufgabe der vorliegenden Erfindung ist daher die Entwicklung eines Verfahrens zum Erkennen der Anwesenheit von Rissen oder abgefasten Rändern auf der Oberfläche von Holzbrettern. Diese Aufgabe wird durch ein Verfahren gemäß dem Anspruch 1 gelöst.

Weitere Vorteile und Merkmale der Erfindung gehen näher aus der folgenden Beschreibung von Beispielen der Erfindung hervor, die richtungsweisend, jedoch nicht begrenzend und mit Bezug auf die beigefügten Zeichnungen dargestellt sind. Es zeigen:
Figur 1a und 1b einen allgemeinen sich im Bereich eines Risses befindlichen Querschnitt eines Holzbrettes, das von abgewandten Seiten von einem ersten bzw. zweiten Lichtbündel beaufschlagt wird,
Figur 2a und 2b das entsprechende Diagramm, das man erhält, indem längs der Breite des Holzbrettes die Lichtstärke erfasst wird, die vom Holzbrett zufolge einer Beleuchtung mit dem ersten bzw. zweiten Lichtbündel in Übereinstimmung mit Figur 1a und 1b zerstreut wird,
Figur 3a und 3b einen allgemeinen sich im Bereich eines abgefasten Randes befindlichen Querschnitt eines Holzbrettes, das von abgewandten Seiten von einem ersten bzw. zweiten Lichtbündel beaufschlagt wird,
Figur 4a und 4b das entsprechende Diagramm, das man erhält, indem längs der Breite des Holzbrettes die Lichtstärke erfasst wird, die vom Holzbrett zufolge seiner Beleuchtung mit dem ersten bzw. zweiten Lichtbündel in Übereinstimmung mit Figur 3a und 3b zerstreut wird,
Figur 5 einen allgemeinen Querschnitt eines Holzbrettes zeigt, dessen zu überprüfender Oberfläche sich auf alle vier Seiten des Holzbrettes erstreckt.

Die Erfindung betrifft ein Verfahren zum Erkennen der Anwesenheit von Fehlern wie Rissen oder abgefasten Rändern auf der Oberfläche von Holzbrettern. Es handelt sich um ein typisches Schrittuntersuchungsverfahren, d.h. um ein Verfahren, bei dem für jeden allgemeinen Querschnitt Si des Holzbrettes dieselbe Verfahrensschritte wiederholt werden, wobei i von 1 bis n wechselt und n seinerseits in Abhängigkeit des Untersuchungsschrittes, den man anwenden will, frei festlegbar ist. Je größer ist n, desto dichter und näher werden untereinander die überprüften Querschnitte Si und desto zuverlässiger wird das Untersuchungsergebnis sein. Die Wahl von n wird daher vom Benutzer des Verfahrens getroffen, der persönlich den notwendigen Kompromiss zwischen der Bedürfnis eine sorgfältige Prüfung des Holzbrettes zu erhalten und der Zeit und den Kosten, welche die Untersuchung einer großen Anzahl von Querschnitten Si verursacht, beurteilen wird.

Für jeden allgemeinen Querschnitt Si werden folgenden Verfahrensschritte durchgeführt:
**a)** der Querschnitt Si wird mit einem ersten, auf die zu prüfender Oberfläche des Querschnittes Si einfallenden Lichtbündel F1i schräg beaufschlagt;
**b)** aus einer Erfassungsstelle T wird Stelle für Stelle die Lichtstärke I1i des Lichtes erfasst, das von der zu überprüfenden Oberfläche zerstreut wird, wenn diese von dem ersten Lichtbündel F1i beaufschlagt wird. Enfindungsgemäss sind folgende weitere Verfahrenschnitte vorgesehen:
**c)** nach Ausschalten des ersten Lichtbündels F1i wird derselbe Querschnitt Si wird mit einem zweiten Lichtbündel F2i schräg beaufschlagt, der von der dem ersten Lichtbündel gegenüberliegenden Seite ausgeht und gleichfalls auf die zu prüfender Oberfläche des Querschnittes Si einfällt;
**d)** aus derselben Erfassungsstelle T wird Stelle für Stelle auch die Lichtstärke I2i des Lichtes erfasst, das von der zu überprüfender Oberfläche zerstreut wird, wenn diese von dem zweiten Lichtbündel F2i beaufschlagt wird;
**e)** Stelle für Stelle wird überprüft, ob die Lichtstärke I1i, die während der Beleuchtung der zu prüfenden Oberfläche des Querschnittes Si mit dem ersten Lichtbündel F1i erfasst wurde, und die Lichtstärke I2i, die während der Beleuchtung der zu prüfenden Oberfläche des Querschnittes Si mit dem zweiten Lichtbündel F2i erfasst wurde, etwa gleich sind, und
**f)** jene Stellen, falls es solche geben sollte, für die die Überprüfung nach dem vorangehenden Schritt e) zu einem negativen Ergebnis geführt hat, werden als zu einem Riss angehörend, wenn sie sich innerhalb der zu überprüfenden Oberfläche befinden, oder als zu einem abgefasten Rand angehörend, wenn sie sich in der Nähe des Randes der zu überprüfenden Oberfläche befinden, bezeichnet, während jene Stellen für die die Überprüfung nach dem vorangehenden Schritt e) zu einem positiven Ergebnis geführt hat, als zu dem Teil der zu überprüfenden Öberfläche angehörend bezeichnet werden, der keine Rissen und abgefasten Rändern aufweist.

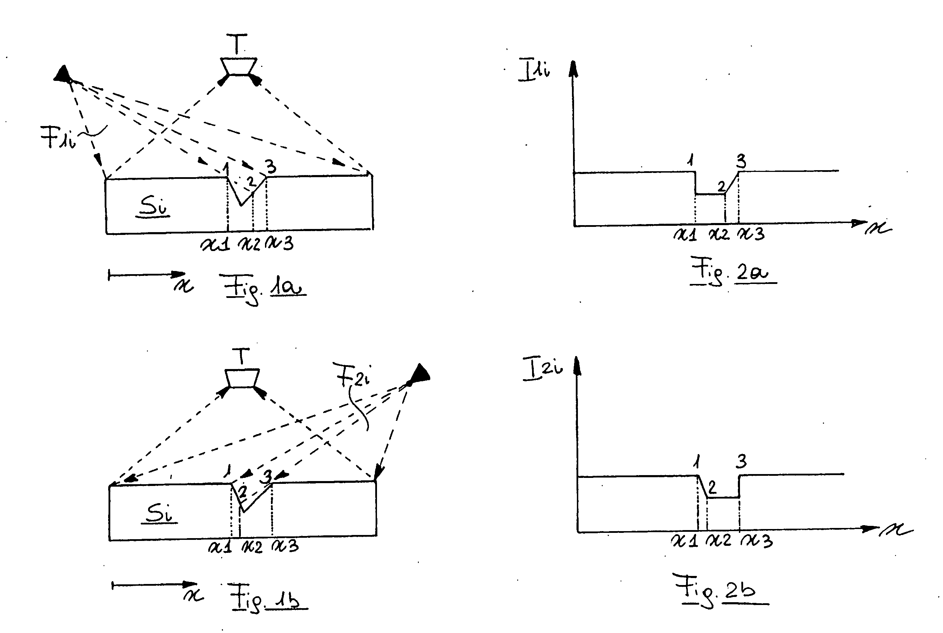

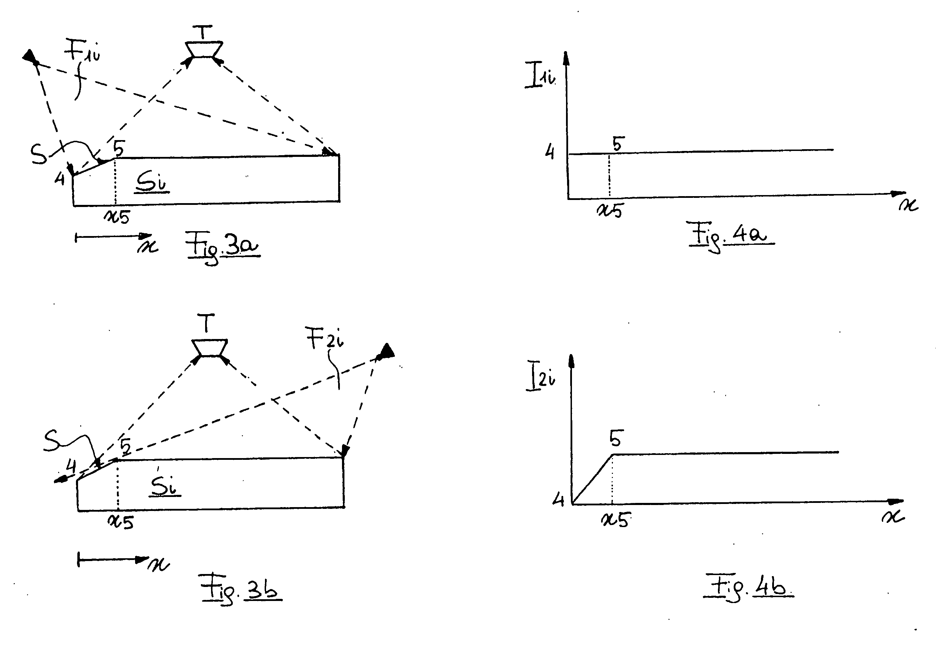

Das erfindungsgemäße Verfahren wird nachfolgend unter Bezugnahme auf zwei typische Situationen näher beschrieben. Die erste Situation bezieht sich auf ein, einen Riss aufweisendes Holzbrett, auf das sich die Figuren 1a bis 2b beziehen. Die zweite Situation bezieht sich auf ein einen abgefasten Rand aufweisendes Holzbrett, auf das sich die Figuren 3a bis 4b beziehen.

Vor allem wird klargestellt, dass die oben genannten Figuren 1a-2b sich auf denselben, allgemeinen Querschnitt Si beziehen, der in verschiedenen Zeitpunkten von zwei Lichtbündeln F1i und F2i beleuchtet wird. Ähnlich beziehen sich auch die oben genannten Figuren 3a-4b auf denselben, allgemeinen Querschnitt Si, der in verschiedenen Zeitpunkten von zwei Lichtbündel F1i und F2i beleuchtet wird. Dies ist typisch für alle Abtastungsverfahren, wie auch jenes, das den Gegenstand der vorliegenden Erfindung bildet, bei denen das Holzbrett längs der eigenen Längsrichtung durch einen Arbeitsbereich vorgeschoben wird, in dem am allgemeinen Querschnitt Si, der sich von Mal zu Mal darin befindet, die Verfahrensschritte durchgeführt werden.

Um zu vermeiden, dass sich die beiden Lichtbündel F1i und F2i miteinander stören, kann die Beleuchtung des allgemeinen Querschnittes Si auf zwei verschiedenen Arten ausgeführt werden.

Eine erste Art sieht vor, dass die Lichtbündel F1i und F2i in derselben Ebene aber in verschiedenen und darauffolgenden Zeitpunkten wirken. Eine zweite Art sieht vor, dass die Lichtbündel F1i und F2i in zwei verschiedenen Ebenen und in verschiedenen Zeitpunkten wirken, wobei sowohl die Ebenen als auch die Zeitpunkte miteinander auf bekannte Art und Weise in Korrelation stehen.

Im ersten Fall wird das längs seiner Längsrichtung vorrückende Holzbrett an regelmäßigen Zeitabschnitten angehalten, die vom gewählten Untersuchungsschritt abhängen. Jedes Mal, dass das Holzbrett stillsteht, wird, zuerst mit dem ersten Lichtbündel F1i von einer Seite aus und danach mit dem zweiten Lichtbündel F2i von der anderen Seite aus, der allgemeine Querschnitt Si beleuchtet, der sich an diesem Zeitpunkt im Bereich der Ebene befindet, in der die beiden Lichtbündel F1i und F2i wirken.

Im zweiten Fall hingegen wird das längs seiner Längsrichtung vorrückende Holzbrett nicht angehalten, sondern an regelmäßigen Zeitabschnitten, die vom Untersuchungsschritt abhängen, mit beiden Lichtbündeln F1i und F2i, beleuchtet, von denen jedes in der eigenen Ebene wirkt. Die beiden Ebenen sind zweckmäßiger Weise in Abhängigkeit des gewählten Untersuchungsschrittes derart voneinander versetzt, dass der allgemeine Querschnitt Si zuerst im Bereich der Ebene beleuchtet wird, wo nur das erste Lichtbündel F1i wirkt, und dann im Bereich der Ebene, wo nur das zweite Lichtbündel F2i wirkt.

Dies vorausgesetzt, wird das Verfahren in Übereinstimmung mit dem ersten Beispiel erläutert, auf das sich die Figuren 1a-2b beziehen.

In diesen wurde der Riss außermaßstäblich übertrieben groß gezeichnet. Mit 1 wurde eine erste Kante des Risses angegeben und mit 3 eine zweite Kante desselben.

Aus Figur 1a und 1b sind die Lichtbündel F1i und F2i, die an verschiedenen Zeitpunkten von geeigneten Lichtquellen, bevorzugter Weise Laserlichtquellen, ausgesandt werden, und die Erfassungsstelle T ersichtlich, die beispielsweise eine Kamera sein könnte, mittels der das von der zu überprüfenden Oberfläche des Querschnittes Si zerstreute Licht erfasst wird, wobei es sich im vorliegenden Fall um die obere Oberfläche handelt.

Jede Stelle der zu überprüfenden Oberfläche des Querschnitts Si zerstreut in Richtung der Kamera T das einfallende Licht des Lichtbündels F1i und des Lichtbündels F2i. Es ist daher möglich, Stelle für Stelle der zu überprüfenden Oberfläche des Querschnitts Si den Wert der zerstreueten Lichtstärken I1i und I2i zu erfassen.

In der Figur 2a ist qualitativ in der Form eines Diagramms der Verlauf der Lichtstärke I1i längs der Stellen der zu überprüfenden Oberfläche des Querschnittes Si in Anwesenheit des Lichtbündels F1i dargestellt, wobei die Abszisse x von Null bis zu dem Wert der der Breite des Querschnittes Si gleich ist wechselt.

Man sieht, dass mit Ausnahme des Bereiches in der Nähe des Risses das Niveau der durch die Kamera T erfassten Lichtstärke I1i etwa konstant für alle Stellen der zu überprüfenden Oberfläche ist.

Beginnend mit der ersten Kante 1 des Risses hat man:
einen ersten rasch bis zu einem Minimum absteigenden Diagrammabschnitt, welcher der Kante 1 des Risses entspricht, bei welcher plötzlich die Beleuchtung mit dem Lichtbündels F1i nicht mehr möglich ist,
einen zweiten kurzen Diagrammabschnitt, in dem die Lichtstärke I1i etwa auf ihrem Minimumwert konstant bleibt, welcher dem Wandabschnitt 1-2 des Risses entspricht, der nicht vom Lichtbündel F1i beleuchtet werden kann und
schließlich einen dritten Diagrammabschnitt allmählich aufsteigend, welcher dem Wandabschnitt 2-3 des Risses entspricht, der bei Zunahme der Abszisse x immer mehr vom Lichtbündel F1i beleuchtet wird, bis zu der Kante 3, wo das fern vom Riss erfasste Niveau der Lichtstärke I1i wieder zurückkehrt, weil ab der Kante 3 bei Zunahme von der Abszisse x das Lichtbündel F1i den Querschnitt Si wieder optimal beleuchten kann.

In der Figur 2b ist qualitativ in der Form eines Diagramms der Verlauf der Lichtstärke I2i längs der Stellen der zu überprüfenden Oberfläche des Querschnittes Si in Anwesenheit des Lichtbündels F2i dargestellt, wobei die Abszisse x wieder von Null bis zu einem Wert der der Breite des Querschnittes Si gleich ist wechselt. Dieses Diagramm wiedergibt jenes aus Figur 2a in dem Teil wo kein Riss vorhanden ist. Im Teil, der hingegen den Riss aufweist, sind die Rollen der Kanten 1 und 3, gegenüber dem was in dem Diagramm 2a bezüglich des Lichtbündels F1i gezeigt ist, umgekehrt.

Nun, beginnend wieder von der Abszisse die der Kante 1 entspricht, hat man nämlich:
einen ersten Diagrammabschnitt allmählich absteigend, welcher dem Wandabschnitt 1-2 des Risses entspricht, der bei Zunahme der Abszisse x immer weniger vom Lichtbündel F2i beleuchtet wird, bis zu dem Punkt 2, wo das erfasste Niveau der Lichtstärke I2i sein Minimum erreicht, weil ab dem Punkt 2 bei Zunahme von der Abszisse x das Lichtbündel F2i den Wandabschnitt 2-3 des Risses nicht mehr beleuchten kann,
dann einen kurzen zweiten Diagrammabschnitt, in dem die Lichtstärke I2i etwa konstant auf ihr Minimumwert bleibt, welcher dem Wandabschnitt 2-3 des Risses entspricht, der nicht vom Lichtbündel F2i beleuchtet werden kann und
schließlich einen dritten rasch bis zu dem fern vom Riss erfasste Niveau der Lichtstärke I2i aufsteigendem Diagrammabschnitt, welcher der Kante 3 des Risses entspricht, bei welcher plötzlich die Beleuchtung mit dem Lichtbündels F1i wieder möglich ist.

Beim Vergleich der beiden Diagramme aus Figur 2a und 2b, erscheint klar, dass nur in Anwesenheit von dem Riss angehörenden Stellen bedeutungsvolle Unterschiede in den Werten der beiden Lichtstärken I1i und I2i erfassbar sind. Diese bedeutungsvollen Unterschiede können als Signal der Anwesenheit eines Risses betrachtet werden, wenn die entsprechenden Werte der Abszisse x, die bekannt sind, angeben, dass man sich innerhalb der zu überprüfenden Oberfläche des Holzbrettes befindet und daher sowohl vom Null-Wert als auch vom Höchstwert, der der Breite des Holzbrettes gleich ist, entfernt sind.

Das Verfahren wird nun nachfolgend in Übereinstimmung mit dem zweiten Beispiel erläutert, auf das sich die Figuren 3a-4b beziehen. In dieser wurde der abgefaste Rand außermaßstäblich übertrieben groß gezeichnet. Mit 4 wurde eine erste Kante und mit 5 eine zweite Kante angegeben.

Aus Figur 3a und 3b sind die Lichtbündel F1i und F2i, die an verschiedenen Zeitpunkten von geeigneten Lichtquellen, bevorzugter Weise Laserlichtquellen, ausgesendet werden, und die Erfassungsstelle T ersichtlich, die beispielsweise eine Kamera sein könnte, mit der das von der zu überprüfenden Oberfläche des Querschnittes Si zerstreute Licht erfasst wird, wobei es sich im vorliegenden Fall um die obere Oberfläche handelt.

Jede Stelle der zu überprüfenden Oberfläche des Querschnittes Si zerstreut in Richtung der Kamera T das einfallende Licht des Lichtbündels F1i und des Lichtbündels F2i. Es ist dann möglich, Stelle für Stelle der Oberfläche des Querschnitts Si den Wert der zerstreuten Lichtstärken I1i und I2i zu erfassen.

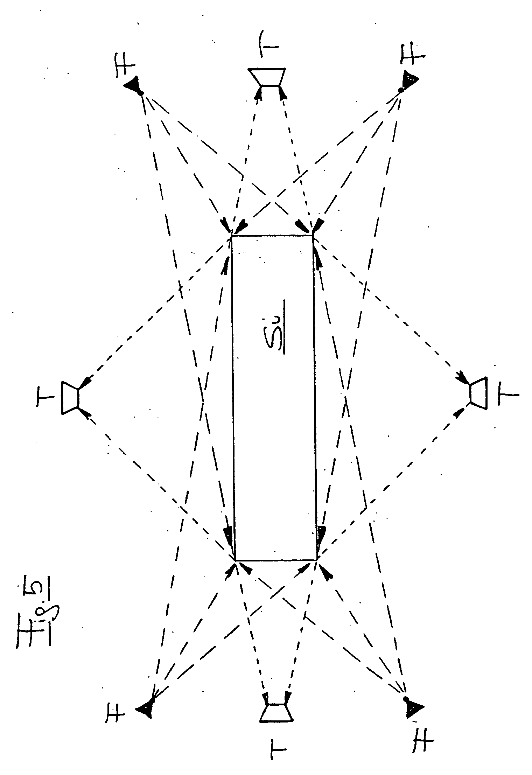

In Figur 4a ist qualitativ in der Form eines Diagramms der Verlauf der Lichtstärke I1i längs der Stellen der zu überprüfenden Oberflächen des Querschnittes Si in Anwesenheit des Lichtbündels F1i dargestellt, wobei die Abszisse x von Null bis zu dem Wert der der Breite des Querschnittes Si gleich ist wechselt.

Man sieht also, dass das Niveau der durch die Kamera T erfasste Lichtstärke I1i etwa konstant für alle Stellen der Oberfläche des allgemeinen Querschnittes Si ist, auch im Endbereiches, wo sich der abgefaste Rand befindet.

In Figur 4b ist qualitativ in der Form eines Diagramms der Verlauf der Lichtstärke I2i längs der Stellen der zu überprüfenden Oberfläche des Querschnittes Si in Anwesenheit des Lichtbündels F2i dargestellt, wobei sich die Abszisse x wieder von Null bis zu einem Wert der der Breite des Querschnittes Si gleich ist wechselt.

Dieses Diagramm wiedergibt jenes aus Figur 4a in dem Teil, wo kein abgefaster Rand vorhanden ist. Im Teil, der hingegen den abgefasten Rand aufweist, kennzeichnet die Abszisse, die der Kante 4 entspricht, den Beginn eines Diagrammabschnittes allmählicher Zunahme der Lichtstärke I2i, die darauf zurückzuführen ist, dass bei Zunahme von der Abszisse x der Abschnitt 4-5 des abgefasten Randes immer mehr vom Lichtbündel F2i beleuchtet wird, bis zu der Kante 5, wo das fern vom abgefasten Rand erfasste Niveau der Lichtstärke I2i erreicht wird.

Beim Vergleich der beiden Diagramme der Figur 4a und 4b erscheint klar, dass nur in Anwesenheit von dem abgefasten Rand angehörenden Stellen bedeutungsvolle Unterschiede in den Werten der beiden Lichtstärken I1i und I2i erfassbar sind. Diese bedeutungsvollen Unterschiede können als Signal der Anwesenheit eines abgefasten Randes betrachtet werden, wenn die entsprechenden, bekannten Werte der Abszisse x angeben, dass man sich in der Nähe des Randes der zu überprüfenden Oberfläche des Holzbrettes befinden, und daher dem Null oder dem Höchstwert der der Breite des Holzbrettes gleich ist sehr nahe liegen.

In den beiden oben angegebenen Beispielen ist die betrachtete, zu überprüfender Oberfläche jene einer einzigen Seite des Holzbrettes. Es ist jedoch auch möglich, eine größere Oberfläche in Betracht zu ziehen. Zum Beispiel kann sich, wie in Figur 5 dargestellt, die zu überprüfender Oberfläche des Querschnittes Si auch auf alle vier Seiten des Umfangs des Holzbrettes erstrecken.

In diesem Fall werden für jede Seite der zu überprüfenden Oberfläche die oben genannten Verfahrensschritte a)-f) durchgeführt, wobei für jede Seite der zu überprüfenden Oberfläche Diagramme erhalten werden, die jenen aus den Figuren 2a, 2b, wenn die Seite einen Riss aufweist, oder jenen aus den Figuren 4a, 4b, wenn die Seite einen abgefasten Rand aufweist, ähnlich sind.

Zu diesem Zwecke wird die Tatsache ausgenützt, dass jede Lichtquelle imstande ist, an verschiedenen Zeitpunkten zwei anliegende Seiten der zu überprüfenden Oberfläche zu beleuchten, sodass insgesamt vier Lichtquellen vorgesehen werden. Jeder Seite wird überdies eine Erfassungsstelle T zugeordnet, um die Lichtstärke des Lichtes zu erfassen, das von der zu überprüfenden Oberfläche zerstreut wird, wobei insgesamt vier verschiedene Erfassungsstellen T vorgesehen werden.

In Figur 5 wurden alle möglichen Lichtbündel F, die man haben kann, zusammen dargestellt sowie das darausentstehende zerstreute Licht, das von den Erfassungsstellen T erfasst wird. Es liegt jedoch nahe, dass von Mal zu Mal ein einziges wirkendes Lichtbündel und eine einzige aktive Erfassungsstelle T vorliegen werden.

Die Feststellung der Anwesenheit von Rissen oder abgefasten Kanten in einer der Seiten der zu überprüfenden Oberfläche erfolgt auf identischer Art und Weise wie unter Bezugnahme auf die beiden oben erläuterten Beispielen erläutert wurde, die sich auf eine aus einer einzigen Seite des Holzbrettes bestehenden zu überprüfender Oberfläche beziehen.

## Patentansprüche

1. Verfahren zum Erkennen der Anwesenheit von Fehlern, wie Rissen oder abgefasten Rändern auf der Oberfläche von Holzbrettern, wobei für jeden allgemeinen Querschnitt (Si) einer Vielzahl (n) von Querschnitten des Holzbrettes die folgenden Verfahrensschritte vorgesehen werden, wobei (i) von 1 bis (n) wechselt und (n) seinerseits in Abhängigkeit des Untersuchungsschrittes den man anwenden will, frei festlegbar ist:
**a)** der Querschnitt (Si) wird mit einem ersten, auf die zu prüfender Oberfläche des Querschnittes (Si) einfallenden Lichtbündel (F1i) schräg beaufschlagt;
**b)** aus einer Erfassungsstelle (T) wird Stelle für Stelle die Lichtstärke (I1i) des Lichtes erfasst, das von der zu überprüfenden Oberfläche zerstreut wird, wenn diese von dem ersten Lichtbündel (F1i) beaufschlagt wird;
**dadurch gekennzeichnet, dass** folgende weitere Verfahrenschritte vorgesehen sind:
**c)** nach Ausschalten des ersten Lichtbündes (F1i) wird derselbe Querschnitt (Si) mit einem zweiten Lichtbündel (F2i) schräg beaufschlagt, der von der dem ersten Lichtbündel gegenüberliegenden Seite ausgeht und gleichfalls auf die zu prüfender Oberfläche des Querschnittes (Si) einfällt;
**d)** aus derselben Erfassungsstelle (T) wird Stelle für Stelle auch die Lichtstärke (I2i) des Lichtes erfasst, das von der zu überprüfenden Oberfläche zerstreut wird, wenn diese von dem zweiten Lichtbündel (F2i) beaufschlagt wird;
**e)** Stelle für Stelle wird überprüft, ob die Lichtstärke (I1i), die während der Beleuchtung der zu prüfenden Oberfläche des Querschnittes (Si) mit dem ersten Lichtbündel (F1i) erfasst wurde, und die Lichtstärke (I2i), die während der Beleuchtung der zu prüfenden Oberfläche des Querschnittes (Si) mit dem zweiten Lichtbündel (F2i) erfasst wurde, etwa gleich sind, und
**f)** jene Stellen, falls es solche geben sollte, für die die Überprüfung nach dem vorangehenden Schritt e) zu einem negativen Ergebnis geführt hat, werden als zu einem Riss angehörend, wenn sie sich innerhalb der zu überprüfenden Oberfläche befinden, oder als zu einem abgefasten Rand angehörend, wenn sie sich in der Nähe des Randes der zu überprüfenden Oberfläche befinden, bezeichnet, während jene Stellen, für die die Überprüfung nach dem vorangehenden Schritt e) zu einem positiven Ergebnis geführt hat, als zu dem Teil der zu überprüfenden Oberfläche angehörend bezeichnet werden, der keine Rissen und abgefasten Ränder aufweist.

2. Verfahren nach Anspruch 1, **dadurch gekennzeichnet, dass** die Lichtbündel (F1i) und (F2i) in voneinander um ein bekanntes Maß versetzten Ebenen wirken.

3. Verfahren nach Anspruch 1, **dadurch gekennzeichnet, dass** die Lichtbündel (F1i) und (F2i) in derselben Ebene wirken.

4. Verfahren nach Anspruch 2 oder 3, **dadurch gekennzeichnet, dass** die zu überprüfender Oberfläche des Querschnitts (Si) sich auf alle vier Seiten des Umfanges desselben erstreckt und dass für jede Seite der zu überprüfenden Oberfläche die oben angeführten Verfahrensschritte a)-f) durchgeführt werden, wobei jedes Lichtbündel zwei anliegende Seiten der zu überprüfenden Oberfläche beleuchtet und die Lichtstärke des von der zu überprüfenden Oberfläche zerstreuten Lichtes von vier verschiedenen Erfassungsstellen (T) erfasst wird, von denen jede der entsprechenden, zugeordneten Seite der zu überprüfenden Oberfläche gewidmet ist.

## Claims

1. Method to assess the presence of defects, such as cracks or bevelled edges, on the surface of wooden boards, in which for each generic cross section (Si) of a plurality (n) of cross sections of the wooden board are provided the following method steps, where (i) varies from 1 to (n) and in turn (n) can be freely determined as a function of the search pitch to be used:
**a)** investing obliquely the cross section (Si) with a first light beam (F1i) incident on the surface of the cross section (Si) to be examined;
**b)** from a detection point (T), measuring point by point the intensity (I1i) of the light diffused by the surface to be examined when it is invested by the first light beam (F1i);
**characterised in that** it comprises the following additional method steps:
**c)** after turning off the first light beam (F1i), investing obliquely the same cross section (Si) with a second light beam (F2i), coming from the opposite part with respect to the first one and also incident on the surface of the cross section (Si) to be examined;
**b)** from the same detection point (T), measuring point by point also the intensity (I2i) of the light diffused by the surface to be examined, when it is invested by the second light beam (F2i);
**e)** checking point by point if the intensity (I1i), which was measured while lighting the surface of the cross section (Si) to be examined with the first light beam (F1i), and the intensity (I2i) that was measured while lighting the surface of the cross section (Si) to be examined with the second light beam (F2i) are about equal, and
**f)** those points, if any, for which the check according to the previous step e) has a negative outcome are classified as belonging to a crack, if they are inside the surface to be examined, or as belonging to a bevelled edge, if they are in proximity to the edge of the surface to be examined, whilst the points for which the check according to the previous phase e) has positive outcome are classified as belonging to the part of the surface to be examined that has no cracks or bevelled edges.

2. Method as claimed in claim 1, **characterised in that** the light beams (F1i) and (F2i) act in planes offset from each other by a known measure.

3. Method as claimed in claim 1, **characterised in that** the light beams (F1i) and (F2i) act in the same plane.

4. Method as claimed in claim 2 or 3, **characterised in that** the surface of the cross section (Si) to be examined extends over all four sides of the perimeter thereof and **in that** for each side of the surface to be examined the aforementioned steps a)-f) are carried out, each light beam illuminating two contiguous sides of the surface to be examined and the intensity of the light diffused by the surface to be examined being measured from four different detection points (T), each of which is dedicated to the respective associated side of the surface to be examined.

## Revendications

1. Procédure pour la détection sur la surface de planches de bois de la présence de défectuosités telles que des fêlures ou des bords chanfreinés, dans laquelle pour chaque section transversale générique (Si) d'une pluralité (n) de sections transversales de la planche de bois sont prévues les suivantes phases de procédure, où (i) varie de 1 à (n) et (n) est à son tour librement déterminable en fonction du pas de mesure que l'on veut utiliser:
**a)** on frappe obliquement la section transversale (Si) avec un premier faisceau lumineux (F1i) incident sur la superficie en examen de la section transversale (Si);
**b)** à partir d'un point de mesure (T) on mesure point par point l'intensité (I1i) de la lumière diffusée par la surface en examen quand cette dernière est frappée par le premier faisceau lumineux (F1i);
**caractérisée par le fait qu'**elle prévoit les ultérieures phases de la procédure suivantes:
**c)** après avoir éteint le premier faisceau lumineux (F1i), on frappe obliquement la même section transversale (Si) avec un second faisceau lumineux (F2i), provenant de la partie opposée par rapport au premier faisceau et incident lui aussi sur la superficie en examen de la section transversale (Si);
**d)** à partir du même point de mesure (T) on mesure point par point même l'intensité (I2i) de la lumière diffusée par la superficie en examen, lorsque celle-ci est frappée par le second faisceau lumineux (F2i);
**e)** on contrôle point par point si l'intensité (I1i), qui a été mesurée pendant l'illumination de la superficie en examen de la section transversale (Si) avec le premier faisceau lumineux (F1i), et l'intensité (I2i), qui a été mesurée pendant l'illumination de la superficie en examen de la section transversale (Si) avec le second faisceau lumineux (F2i), sont plus ou moins égales, et
**f)** ces points, s'ils existent, pour lesquels le contrôle selon la précédente phase e) présente un résultat négatif, se classent comme appartenant à une fêlure, s'ils se trouvent à l'intérieur de la surface en examen, ou comme appartenant à un bord chanfreiné, s'ils se trouvent à proximité du bord de la superficie en examen, alors que les points pour lesquels le contrôle selon la précédente phase e) présente un résultat positif se classent comme appartenant à la partie de superficie en examen qui ne présente par de fêlures ou bords chanfreinés.

2. Procédure selon la revendication 1, **caractérisée en ce que** les faisceaux lumineux (F1i) et (F2i) agissent à des niveaux décalés entre eux selon un mesure connue.

3. Procédure selon la revendication 1, **caractérisé en ce que** les faisceaux lumineux (F1i) et (F2i) agissent dans le même plan.

4. Procédure selon la revendication 2 ou 3, **caractérisée en ce que** la superficie en examen de la section transversale (Si) s'étend sur tous les quattre côtés du périmètre de cette dernière, et **en ce que** pour chaque côté de la superficie en examen on effectue les phases a)-f) citées ci-dessus, chaque faisceau lumineux illuminant deux côtés contigus de la superficie en examen et l'intensité de la lumière diffusée par la superficie en examen étant mesurée par quattre points différents de mesure (T), chacun desquels étant dédié au côté associé respectif de la superficie en examen.
